# EUROPÄISCHE PATENTSCHRIFT

(11) **EP 3 122 418 B1**
(45) Veröffentlichungstag und Bekanntmachung des Hinweises auf die Patenterteilung: **05.10.2022**
(21) Anmeldenummer: 15712605.3
(22) Anmeldetag: 25.03.2015
(51) Int. Cl.: A61N 1/36

(54) **NERVENSTIMULATIONSGERÄT**
NERVE STIMULATION APPLIANCE
APPAREIL DE STIMULATION NERVEUSE

(30) Priorität: 28.03.2014 DE 102014104414
(43) Veröffentlichungstag der Anmeldung: 01.02.2017
(73) Patentinhaber: Mäusl, Roland, 80331 München (DE); Mück, Andreas, 82487 Oberammergau (DE)
(72) Erfinder: Mäusl, Roland, 80331 München (DE); Mück, Andreas, 82487 Oberammergau (DE)
(74) Vertreter: Westphal, Mussgnug & Partner, Patentanwälte mbB
(86) Internationale Anmeldenummer: PCT/EP2015/056389
(87) Internationale Veröffentlichungsnummer: WO 2015/144758

(56) Entgegenhaltungen:
- WO-A1-03/018113
- US-A1- 2006 106 441
- US-A1- 2006 195 170
- US-A1- 2009 187 223
- US-A1- 2013 296 975

## Beschreibung

Die vorliegende Offenbarung betrifft ein nicht-invasives (non-invasives) Nervenstimulationsgerät, das insbesondere verwendet werden kann zur non-invasiven Einflussnahme auf den Nervus Vagus und mit diesem in Wechselwirkung stehender Teilbereiche des Gehirns sowie damit verbundener Organe und Extremitäten, und damit zur Behandlung von psychisch bedingten Krankheiten, von physisch und psychisch bedingten chronischen Schmerzerkrankungen. Psychisch bedingte Krankheiten sind beispielsweise Depressionen, Ängste, Manien, Burnout, Magersucht, Schizophrenien und andere Zwangserkrankungen, aus denen physisch und psychisch bedingte Erkrankungen entstehen können.

Eine Einflussnahme auf den Nervus Vagus mittels Gleichstrom oder Wechselstrom kann Auswirkungen auf physische oder psychische Krankheitszustände haben. Zur Behandlung von Epilepsien ist es bekannt, Patienten operativ - und damit invasiv - einen Schrittmacher zu implantieren, der Stromimpulse an den Nervus Vagus abgibt. Verbunden hiermit sind das Operationsrisiko für den Patienten und relativ hohe Kosten.

Außerdem wird die Art und die Stärke der durch den Schrittmacher abgegebenen Stromimpulse während der Operation eingestellt, und kann damit nach der Implantation des Schrittmachers nicht ohne weiteres und nicht beliebig eingestellt werden. Eine Einstellung der Stromimpulse erfolgt beispielsweise auf Basis während der Operation induzierter Muskelkontraktionen, die allerdings elektrophysikalisch nicht uneingeschränkt sinnvoll sind. Positive und festgestellte Auswirkungen auf psychische Befindlichkeiten, wie zum Beispiel eine Stimmungsaufhellung bei Depressionen, sind damit zufällig und nicht gezielt reproduzierbar. Zudem werden verschiedene Faserarten innerhalb des Nervus Vagus angesprochen, Auswirkungen auf psychische Zustände sind rein zufällig.

Zur Migränebehandlung ist es bekannt, den Nervus Vagus durch ein elektromagnetisches beziehungsweise ein elektrisches Feld zu beeinflussen, das durch ein portables Gerät erzeugt wird. Bei dieser bekannten Stimulation ist das Ziel das Ansprechen einer bestimmten afferenten Faserart des Nervus Vagus, die nur teilweise bezüglich psychosomatischer Symptome Relevanz hat. Der Nervus Vagus führt drei verschiedene Faserarten mit unterschiedlichen Widerständen, Leitungsrichtungen und Leitungsgeschwindigkeiten, wobei es wesentlich ist, die für eine Einflussnahme relevanten Faserarten anzusprechen.

Die US 2013/0296975 A1 beschreibt ein Nervenstimulationsgerät, das dazu ausgebildet ist Stimulationsimpulse in einer Nervenfaser eines Menschen zu erzeugen, die sich in entgegen gesetzten Richtungen der Nervenfaser ausbreiten. Die Stimulationsimpulse, die sich in einer Richtung der Nervenfaser weg vom Gehirn (kaudal) ausbreiten, sollen hierbei schmerzlindernd wirken. Die Stimulationsimpulse, die sich in der Nervenfaser in Richtung des Gehirns ausbreiten, können, beispielsweise durch ein Sinussignal, moduliert oder blockiert werden.

Die WO 03/018113 A1 beschreibt ein Nervenstimulationsgerät, das dazu ausgebildet ist, Aktionspotenziale in einem Nerv auszulösen, die sich in einer ersten Richtung in dem Nerv ausbreiten. Das Nervenstimulationsgerät ist außerdem dazu ausgebildet, Aktionspotenziale zu unterdrücken, die sich in einer entgegengesetzten zweiten Richtung ausbreiten.

Die Erfindung wird durch die Ansprüche definiert. Ausführungsbeispiele werden nachfolgend anhand von Zeichnungen näher erläutert. Die Zeichnungen dienen zur Veranschaulichung des Grundprinzips, so dass nur solche Aspekte, die zum Verständnis des Grundprinzips notwendig sind, dargestellt sind. Die Zeichnungen sind nicht maßstabsgerecht. In den Zeichnungen bezeichnen, sofern nichts anderes angegeben ist, gleiche Bezugszeichen gleiche Merkmale mit gleicher Bedeutung.
- Figur 1: zeigt ein Blockschaltbild eines Nervenstimulationsgeräts;
- Figur 2: zeigt schematisch eine Nervenfaser während einer Stimulation;
- Figur 3: zeigt ein Blockschaltbild eines (nicht anspruchsgemäßen) Nervenstimulationsgeräts mit einem ersten und einem zweiten Stimulator, die jeweils ein Elektrodenpaar aufweisen;
- Figur 4: zeigt ein (nicht anspruchsgemäßes) Ausführungsbeispiel eines Nervenstimulationsgeräts im Detail und veranschaulicht dessen Funktionsweise;
- Figur 5: zeigt eine (anspruchsgemäße) Abwandlung des Nervenstimulationsgeräts gemäß Figur 4;
- Figur 6: zeigt eine weitere (anspruchsgemäße) Abwandlung des Nervenstimulationsgeräts gemäß Figur 4;
- Figur 7: zeigt einen Zeitverlauf eines Stimulationssignals, das wenigstens einen rechteckförmigen Stimulationsimpuls aufweist;
- Figur 8: zeigt einen Zeitverlauf eines Stimulationssignals, das wenigstens einen oszillierenden Stimulationsimpuls mit einer rechteckförmigen Einhüllenden aufweist;
- Figur 9: zeigt einen Zeitverlauf eines Stimulationssignals, das wenigstens einen oszillierenden Stimulationsimpuls mit einer sinusförmigen Einhüllenden aufweist;

- Figur 10: zeigt ein Beispiel eines Gehäuses eines Nervenstimulationsgeräts;
- Figur 11: zeigt ein Beispiel eines (nicht anspruchsgemäßen) Nervenstimulationsgeräts mit einem ersten und einem zweiten Stimulator, die jeweils einen Elektromagnet aufweisen;
- Figur 12: zeigt eine (nicht anspruchsgemäße) Abwandlung des Nervenstimulationsgeräts gemäß Figur 11;
- Figur 13: zeigt eine weitere (nicht anspruchsgemäße) Abwandlung des Nervenstimulationsgeräts gemäß Figur 11.

Zum besseren Verständnis der Funktionsweise der nachfolgend beschriebenen Nervenstimulationsgeräte sind einige neurologische und neurophysiologische Mechanismen zuvor kurz erläutert.

In der Wissenschaft und Medizin ist bekannt, dass ein Großteil menschlicher Handlungen von sogenannten Emotionen beeinflusst bzw. gesteuert wird. Fast alle Emotionen sind für Menschen und andere Säugetiere physisch spürbar, was von der Natur gewollt ist und im Zuge der Evolution eine wichtige Rolle gespielt hat. Eine vom Gehirn als bedrohlich bewertete Situation, Person oder Sache kann physisch spürbare Reaktionen hervorrufen, die von der Natur als Impuls für Flucht, Ekel, Lust oder Verteidigung vorgesehen sind und damit für Überleben und Fortpflanzung eine Rolle spielen kann. Hintergrund ist die Tatsache, dass das limbische System bei Situationen, die als bedrohlich bewertet werden, Teile des Gehirns abschaltet bzw. überbrückt, um wertvolle Reaktionszeit zu "sparen", die durch Nachdenken und eine eventuelle Fehlbewertung verbraucht würde. Hormonelle Reaktionen, wie eine Adrenalinausschüttung oder ähnliches, spielen hierbei nur eine ergänzende Rolle.

Wird in einer Gefahrensituation beispielsweise der Herzschlag erhöht, geschieht das unter anderem über Impulse, die vom Gehirn über den Nervus Vagus an das Herz übertragen werden. Eine wichtige Rolle spielt hier die Tatsache, dass vor allem im Bereich der rechten Herzkammer Neuronen zu finden sind, die Verbindungen zum Nervus Vagus aufweisen, obwohl neuronale Verbindungen sonst nur im Gehirn zu finden sein sollten. Da vor allem Schmerz ein starker Impuls ist, wird er auch als Emotion benutzt. Von der Natur ist eine schnelle Reaktion auf bedrohliche Gegebenheiten vorgesehen, ohne Zeit für eine bewusste Bewertung der Situation durch Nachdenken zu vergeuden. Deshalb laufen viele Emotionen schnell und unbewusst ab, ohne eine Möglichkeit zu geben, rational, d.h. durch Vernunft die Situation zu bewerten oder auf Reaktionen Einfluss nehmen zu können. Die Verknüpfung dieser Umstände mit physisch spürbaren Emotionen stellt somit die effektivste Methode dar, Menschen und Säugetiere zu einer schnellen und zuverlässigen Reaktion zu bewegen.

Zur Verdeutlichung sei ein Beispiel aus dem Bereich der Nahrungsaufnahme erläutert: Das bloße Wissen, dass eine Speise ungesund ist, weil sie beispielsweise viel Zucker, Fett, Zusatzstoffe usw. enthält, hält nicht zwangsläufig davon ab, sie doch zu testen oder dauerhaft zu konsumieren. Wenn die Speise jedoch physisch spürbare Emotionen in Form von Ekel, einem Würgereiz oder einem flauen Gefühl im Magen verursacht, wird man abgeneigt sein, diese Speise zu sich zu nehmen. Trennungsschmerz und Schmetterlinge im Bauch sind weitere Beispiele von physisch spürbaren Emotionen.

Problematisch ist die Tatsache, dass das Gehirn bezüglich des Abspeicherns von Handlungsmustern schlecht unterscheiden kann zwischen Realität und Gedachtem. Daraus resultieren die erwiesene Effektivität von autogenem Training oder auch die Tatsache, dass Menschen Phobien bezüglich Situationen entwickeln können, die sie selbst nie erlebt und rational bewertet haben, von denen sie aber gelesen oder sie anschließend durchdacht haben. Diese Gedankenbilder, wie z.B. von einem ein Flugzeugabsturz, sind für das Gehirn unter Umstanden ebenso real wie eine tatsächlich durchlebte Situation. Auf diesem Weg entwickelt das Gehirn sogenannte Trigger, denen bestimmte Handlungsmuster zugeordnet werden. Je öfter diese Trigger aktiviert werden, desto mehr Handlungsmuster werden damit verknüpft. Sind die Trigger auf negativ bewertete Umstände bezogen, wie beispielsweise ein Flugzeugabsturz, nach eigenem Empfinden dick machende Nahrung, oder ein unangenehmes Arbeitsumfeld, werden automatisch negative Emotionen als Handlungsverstärker mit ihnen verknüpft.

Damit ist es mit den heute vorhandenen Informationsmöglichkeiten sehr einfach, emotionsunterstützte Verhaltensmuster aufzubauen. Entwickelt eine Person beispielsweise eine Magersucht, sind mit dem Trigger "Nahrungsmittel" mehrere Gedankenmuster verknüpft, die eine emotional unterstützte Reaktion hervorrufen, wie z.B.: Nahrungsmittel machen dick, Dicke sind hässlich, Hässliche werden verspottet, Verspottete sind ausgegrenzt, usw.. Auch andere Faktoren, wie beispielsweise der Arbeitsplatz, Lebensumstände, Gegenstände, Lebewesen (wie z.B. Mäuse, Spinnen, Ratten, Schlangen, etc.) können als Trigger dienen. Wird ein Trigger nicht bewusst rational bewertet und beispielsweise als negativ eingestuft, bilden sich immer mehr emotionsgestützte Denkmuster als Verknüpfungen und damit verbundene Handlungsweisen.

Gegen eine unbekannte Anzahl N an emotionsgestützten Verhaltensmustern ist ab einer gewissen Anzahl mit einer konventionellen Gesprächstherapie oder gar aus eigener Kraft kaum mehr anzugehen. Erstens müsste hierfür jedes dieser Muster bekannt sein, zweitens müsste für jedes dieser Muster ein Gegenargument gefunden werden und drittens müsste jedes dieser Gegenargumente entgegen der eigenen Empfindungen als das Richtige bewertet werden, um synaptische Verbindungen zum Trigger aufzubauen, die zahlreicher und besser vernetzt sind, als es bei den die nichterwünschten Mustern der Fall ist.

Da es psychotherapeutisch, medikamentös oder technisch kaum möglich ist, auf eine komplette Handlungskette im Gehirn gezielt Einfluss zu nehmen und auch noch nicht alle Zusammenhänge vollständig erforscht und bekannt sind, ist es nur begrenzt wirkungsvoll, mit konventionellen Methoden (psychologisch und medikamentös) psychische Erkrankungen zu behandeln, die durch emotionsunterstützte Verhaltensmuster ausgelöst werden. Medikamentengestützte Therapien wirken zu großflächig, um als zufriedenstellende Lösung betrachtet werden zu können. Da aber physisch spürbare Emotionen von der Natur als Handlungsverstärker eingesetzt werden, kann es wirkungsvoll sein, diese physisch spürbaren Emotionen zu verringern oder vollständig abzuschalten. Wird ein Denkmuster auf einen Trigger nicht mehr emotional, d.h. physisch spürbar unterstützt, ist es leichter möglich, den Trigger rational und damit mit höherer Wahrscheinlichkeit korrekt zu bewerten.

Nimmt man beispielsweise einer magersüchtigen Person das physisch spürbare Gefühl von Angst, Panik und Ekel bei dem Gedanken an Nahrungsmittel und den damit verbundenen körperlichen Befindlichkeiten, so gibt man der Person die Möglichkeit, den Trigger Nahrungsmittel der Realität entsprechend zu bewerten und neue neuronale Verbindungen aufzubauen, die den Trigger positiv bewerten und alte zu kappen. In weiteren Schritten hat sie sogar die Möglichkeit, Neuronen eine neue Funktion zuzuweisen, anstatt nur die synaptischen Verbindungen zu kappen, was eine dauerhafte Beseitigung der psychischen Erkrankung bedeuten würde.

Physisch spürbare Emotionen wie "Schmetterlinge im Bauch", das "gebrochene Herz", etc. werden durch elektrische Gehirnimpulse generiert und hauptsächlich durch efferente Fasern einer bestimmten Faserart des Nervus Vagus übertragen. Solche Emotionen werden durch eine Feedbackschleife, die über afferente Fasern des Nervus Vagus zurück in das Gehirn verläuft, wahrgenommen.

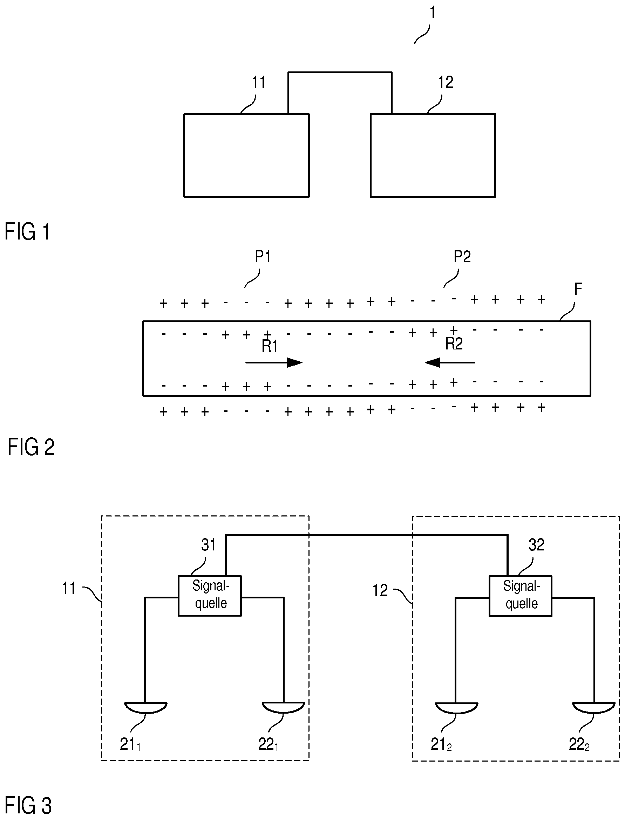

Figur 1 zeigt schematisch ein Nervenstimulationsgerät 1 gemäß einem Ausführungsbeispiel der Erfindung. Dieses Nervenstimulationsgerät 1 umfasst einen ersten Stimulator 11 und einen zweiten Stimulator 12. Der erste Stimulator 11 ist dazu ausgebildet, einen ersten Reiz in einer Nervenfaser eines Nervs eines Menschen oder eines Säugetiers auszulösen, der sich in einer ersten Richtung in der Nervenfaser ausbreitet, und der zweite Stimulator 12 ist dazu ausgebildet, einen zweiten Reiz in der Nervenfaser auszulösen, der sich in einer zweiten Richtung, die der ersten Richtung entgegengesetzt ist, in der Nervenfaser ausbreitet. Der erste Stimulator 11 und der zweite Stimulator 12 sind derart synchronisiert, dass der erste Reiz und der zweite Reiz innerhalb eines vorgegebenen Zeitfensters erzeugt werden. Diese Funktionsweise des Nervenstimulationsgeräts ist nachfolgend anhand von Figur 2 erläutert.

Figur 2 zeigt schematisch eine Nervenfaser eines Nervs während einer Stimulation. Unter "Nerv" ist nachfolgend ein Nerv eines Menschen oder eines Säugetiers zu verstehen. Ein solcher Nerv ist beispielsweise der Vagusnerv (Nervus Vagus) des Menschen. Die Nervenfaser ist beispielsweise eine sogenannte C-Faser des Vagusnervs.

Im Ruhezustand der Nervenfaser F ist zwischen einem Äußeren der Nervenfaser F und einem Inneren der Nervenfaser F ein Potentialunterschied vorhanden, der bei der C-Faser des Vagusnervs circa -70 mV (Mikrovolt) zum Nullbetrag beträgt. Das elektrische Potential im Inneren der Nervenzelle ist dabei negativ im Vergleich zum elektrischen Potential außerhalb der Nervenzelle. Verursacht wird diese Potentialdifferenz durch positiv geladene Natriumionen, die außerhalb der Nervenfaser (der Nervenzelle) F angeordnet sind und negativ geladene Kaliumionen, die innerhalb der Nervenfaser angeordnet sind. Die positiv geladenen Natriumionen können jedoch über Kanäle in das Innere der Nervenfaser gelangen. Durch Anlegen eines geeigneten Stimulationsimpulses an die Nervenfaser F kann das elektrische Potential der Nervenfaser F gegenüber dem oben erläuterten Ruhepotential verschoben werden. Man spricht in diesem Fall von einer Depolarisation. Eine solche Depolarisation, die durch das Einfließen von Natriumionen in das Innere der Nervenfaser F hervorgerufen wird, breitet sich wie eine Welle in Längsrichtung der Nervenfaser F aus, das heißt die Polarisation klingt in solchen Bereichen, in denen sie zuerst erfolgt ist, wieder ab, erfasst in der Richtung, in der sich die Depolarisation ausbreitet, jedoch weitere Bereiche der Nervenfaser. Die Ausbreitungsgeschwindigkeit beträgt beispielsweise zwischen 1 m/s und 30 m/s.

Die Anregung einer solchen Depolarisation in der Nervenfaser F wird nachfolgend als Erregung oder als Reiz bezeichnet. Die Reizausbreitung in der Nervenfaser F funktioniert nach dem "Alles-oder-Nichts-Prinzip". Das heißt, ein einmal ausgelöster Reiz breitet sich in der Nervenfaser F entweder bis zum Ziel aus oder bis er durch eine andere Depolarisation gestoppt wird. Das "Ziel" ist für einen Reiz, der sich von einem inneren Organ, wie beispielsweise dem Magen, kommend ausbreitet, immer das Gehirn.

Die durch das Nervenstimulationsgerät 1 ausgelösten, sich in entgegengesetzter Richtung ausbreitenden ersten und zweiten Reize blockieren sich gegenseitig, das heißt der erste Reiz kann sich nicht über die Position hinaus ausbreiten, an der der zweite Reiz erzeugt wird, und der zweite Reiz kann sich nicht über die Position der Nervenfaser F hinaus ausbreiten, an der der erste Reiz erzeugt wird. In Figur 2 ist schematisch eine erste Position P1 dargestellt, an der ein erster Reiz (Erregung, Depolarisation) ausgelöst wird, der sich in einer ersten Richtung R1 der Nervenfaser F ausbreitet, und P2 bezeichnet eine beabstandet zu der ersten Position P1 angeordnete Position P2, an der ein zweiter Reiz ausgelöst wird, der sich in einer zweiten Richtung R2 ausbreitet, die entgegengesetzt zu der ersten Richtung R1 verläuft. Die Richtung der Reizausbreitung kann über die Art des Stimulationsimpulses eingestellt werden. Dies ist nachfolgend anhand verschiedener Ausführungsbeispiele noch im Detail erläutert.

Die beiden Reize blockieren sich jedoch nicht nur gegenseitig, sondern es werden damit auch Reize blockiert, die sich von einem inneren Organ oder einer Extremität kommend in Richtung des Gehirns ausbreiten, oder umgekehrt. Ziel ist es, die Reizweiterleitung lediglich zu verhindern. Die beispielsweise verwendeten Ströme sind vergleichsweise gering und es besteht keine Gefahr von physischen Beeinträchtigungen oder gar ernstzunehmenden Verletzungen.

Für die Erläuterung sei angenommen, dass der erste Stimulator 11 für die Reizerzeugung an der ersten Position P1 verantwortlich ist und dass der zweite Stimulator 12 für die Reizerzeugung an der zweiten Position P2 verantwortlich ist. Die Synchronisation des ersten Stimulators 11 und des zweiten Stimulators 12 ist davon abhängig, wie weit diese beiden Positionen P1, P2 voneinander entfernt sind und wie schnell die Reizausbreitung innerhalb der Nervenfaser F erfolgt. Allgemein gilt, dass ein Reiz an einer der beiden Positionen P1, P2 erzeugt werden sollte, bevor der an der anderen der beiden Positionen P1, P2 erzeugte Reiz die eine der beiden Positionen P1, P2 erreicht hat. Bei einem Ausführungsbeispiel ist vorgesehen, dass die Reize an der ersten und zweiten Position P1, P2 wenigstens annäherungsweise gleichzeitig erzeugt werden.

Die Reize können elektrisch, also durch Einprägen eines Stroms in die Nervenfaser F, elektrostatisch, also durch Anlegen eines elektrischen Feldes an die Nervenfaser F, oder elektromagnetisch, also durch Anlegen eines Magnetfelds an die Nervenfaser F ausgelöst werden. Gemäß einem Ausführungsbeispiel ist das Nervenstimulationsgerät 1 so ausgestaltet, dass die beiden Stimulatoren 11, 12 die ersten und zweiten Reize jeweils auf gleiche Weise erzeugen. Gemäß einem weiteren Ausführungsbeispiel sind die Stimulatoren 11, 12 so ausgestaltet, dass sie die beiden Reize auf unterschiedliche Art und Weise erzeugen.

Figur 3 veranschaulicht ein Nervenstimulationsgerät 1, das einen ersten Stimulator 11 und einen zweiten Stimulator 12 aufweist. Bei diesem Ausführungsbeispiel umfasst jeder der beiden Stimulatoren 11, 12 ein Elektrodenpaar mit einer ersten Elektrode 21₁, 22₁ und einer zweite Elektrode 21₂, 22₂. Außerdem umfasst jeder der beiden Stimulatoren 11, 12 eine Signalquelle 31, 32, die an das Elektrodenpaar des jeweiligen Stimulators 11, 12 angeschlossen ist. Jede der beiden Signalquellen 31, 32 ist dazu ausgebildet, ein Stimulationssignal, das wenigstens einen Stimulationsimpuls aufweist, an das an die jeweilige Signalquelle 31, 32 angeschlossene Elektrodenpaar abzugeben. Diese Stimulationsimpulse werden in noch zu erläuternder Weise über die Elektroden in die Nervenfaser F eingekoppelt, so dass jedes Elektrodenpaar mit der ersten Elektrode 21₁, 22₁ und der zweiten Elektrode 21₂, 22₂ eines Stimulators 11, 12 dazu ausgebildet ist, einen der zuvor erläuterten Reize in der Nervenfaser F zu erzeugen, wenn die Elektrodenpaare in noch zu erläuternder Weise in die Nähe der Nervenfaser F gebracht werden.

Die erste Signalquelle 31 und die zweite Signalquelle 32 sind derart synchronisiert, dass die durch die Stimulationsimpulse hervorgerufenen Reize wie oben erläutert synchron erzeugt werden.

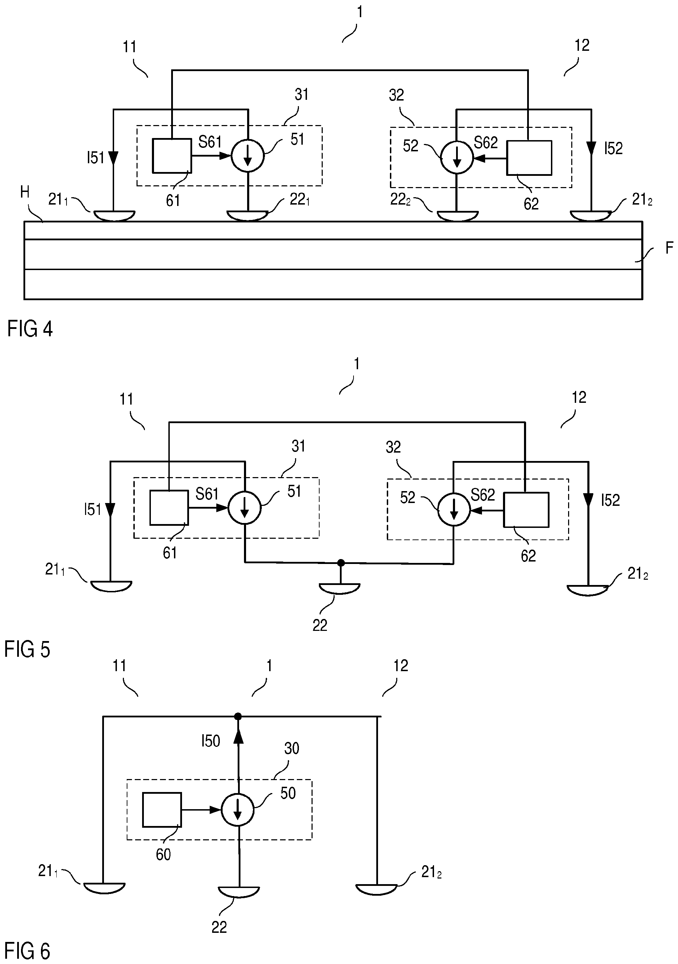

Gemäß einem Ausführungsbeispiel umfasst jede der beiden Signalquellen 31, 32 eine gesteuerte Stromquelle und die Elektrodenpaare 21₁ 22₁ bzw. 21₂, 22₂ der beiden Stimulatoren 11, 12 besitzen jeweils elektrisch leitende Kontaktflächen. Ein Ausführungsbeispiel eines solchen Nervenstimulationsgeräts 1 ist in Figur 4 dargestellt.

Bei dem in Figur 4 dargestellten Nervenstimulationsgerät 1 umfasst jede der beiden Signalquellen 31, 32 eine steuerbare Stromquelle 51, 52 und eine die steuerbare Stromquelle 51, 52 steuernde Ansteuerschaltung 61, 62. Jede der Stromquellen 51, 52 ist dazu ausgebildet, nach Maßgabe eines von der zugehörenden Steuerschaltung 61, 62 gelieferten Steuersignals S61, S62 den Strom I51, I52 an das jeweilige Elektrodenpaar 21₁, 22₁ bzw. 21₂, 22₂ abzugeben. Das von jeder Signalquelle 31, 32 bei diesem Ausführungsbeispiel abgegebene Stimulationssignal ist somit ein Stromsignal und die Stimulationsimpulse sind Stromimpulse.

Figur 4 zeigt das Nervenstimulationsgerät 1 während der Anwendung. Hierzu werden die Elektrodenpaare 21₁, 22₁ bzw. 21₂, 22₂ der beiden Stimulatoren 11, 12 in der Nähe der zu beeinflussenden Nervenfaser F in Kontakt mit der Haut H einer zu behandelnden Person gebracht. Die Haut H und die Nervenfaser F sind in Figur 4 schematisch dargestellt. Die Elektrodenpaare 21₁, 22₁ bzw. 21₂, 22₂ werden hierbei so in Kontakt mit der Haut H gebracht, dass die vier Elektroden der Elektrodenpaare in Längsrichtung der zu beeinflussenden Nervenfaser F im Wesentlichen auf einer Linie liegen. Die Funktionsweise des in Figur 4 dargestellten Nervenstimulationsgeräts 1 ist nachfolgend erläutert.

Für die Erläuterung sei zunächst der erste Stimulator 11 mit der ersten Signalquelle 31 und dem Elektrodenpaar 21₁, 22₁ betrachtet. Zur Erzeugung eines Stimulationsimpulses steuert die Steuerquelle 61 die steuerbare Stromquelle 51 derart an, dass die Stromquelle 51 einen Stromimpuls an das Elektrodenpaar 21₁, 22₁ abgibt. Unter einem "Stromimpuls" ist in diesem Zusammenhang ein für eine vorgegebene Zeitdauer fließender Strom zu verstehen, der eine definierte Stromstärke (Amplitude) besitzt. Die Zeitdauer, für welche dieser Strom fließt wird nachfolgend als Dauer des Stromimpulses bezeichnet. Die Stromstärke kann während der Dauer des Stromimpulses wenigstens annähernd konstant bleiben oder die Stromstärke kann während der Dauer des Stromimpulses variieren. Dies ist nachfolgend noch im Detail erläutert. Während des Stromimpulses fließt der Strom I51 der Stromquelle 51 über eine der Elektroden des Elektrodenpaars, wie beispielsweise die erste Elektrode 21₁, die Haut H, das unter der Haut H liegende Gewebe und die Nervenfaser F und die andere der beiden Elektroden, wie beispielsweise die zweite Elektrode 22₁, und wieder zurück zur Stromquelle 51. Der durch die Nervenfaser F oder zumindest in der Nähe der Nervenfaser F durch das umliegende Gewebe fließende Strom I51 bewirkt eine Depolarisation der Nervenfaser F und damit das Auslösen eines Reizes in der Nervenfaser F. Dieser Reiz breitet sich in der Nervenfaser F ausgehend von einer Position unterhalb der ersten Elektrode 21₁ über eine Position unterhalb der zweiten Elektrode 22₁ hinaus weiter aus, und zwar in Richtung unterhalb der Positionen, an denen die erste und zweite Elektrode 21₂, 22₂ des Elektrodenpaars des zweiten Stimulators 12 an der Haut H anliegen.

Dieses Elektrodenpaar 21₂, 22₂ des zweiten Stimulators 12 ist in entsprechender Weise wie das Elektrodenpaar des ersten Stimulators 11 in der Lage, einen Reiz in der Nervenfaser F auszulösen, indem die steuerbare Stromquelle 52 während der Dauer eines Stimulationsimpulses einen Strom I52 erzeugt, der über eine der beiden Elektroden, wie beispielsweise die erste Elektrode 21₂, in die Haut H, das darunterliegende Gewebe und die Nervenfaser F und über die andere Elektrode des Elektrodenpaars, wie beispielsweise die zweite Elektrode 22₂, zur Stromquelle 52 zurückfließt. Der durch diesen Strom I52 in der Nervenfaser F ausgelöste Reiz wird derart erzeugt, dass sich dieser Reiz in einer Richtung der Nervenfaser F ausbreitet, die entgegengesetzt zu der Richtung verläuft, in der sich der Reiz ausbreitet, der durch den ersten Stimulator 11 ausgelöst wird.

Die Richtung der Reizausbreitung ist abhängig von der Richtung, in der der Strom während des Stromimpulses durch die Nervenfaser F bzw. das umliegende Gewebe fließt. Die physikalische Stromrichtung dieses Stromes, also die Richtung, in der der Elektronenfluss stattfindet, entspricht dabei der Richtung der Reizausbreitung. Die Elektrodenpaare 21₁, 22₁ bzw. 21₂, 22₂ sind also so gewählt, dass die durch diese Elektrodenpaare in die Nervenfaser F oder das umliegende Gewebe eingeprägten Ströme I51, I52 in entgegengesetzten Richtungen durch die Nervenfaser bzw. das umliegende Gewebe verlaufen, wobei die Elektrodenpaare so angeordnet sind, dass das zweite Elektrodenpaar in Richtung des durch das erste Elektrodenpaar eingeprägten Stroms vor dem ersten Elektrodenpaar liegt, bzw. dass das erste Elektrodenpaar in Richtung des durch das zweite Elektrodenpaar eingeprägten Stroms vor dem zweiten Elektrodenpaar liegt. In dem dargestellten Ausführungsbeispiel, in dem die Ströme I51, I52 über die erste Elektrode 21₁ über die Haut H in das darunterliegende Gewebe und die Nervenfaser F eingebracht werden, liegen die zweiten Elektroden 22₁, 22₂ räumlich zwischen den ersten Elektroden 21₁, 21₂. Außerdem ist die zweite Elektrode 22₁ des ersten Elektrodenpaars näher an der ersten Elektrode 21₁ des ersten Elektrodenpaars als an der ersten Elektrode 21₂ des zweiten Elektrodenpaars angeordnet. Entsprechend ist die zweite Elektrode 22₂ des zweiten Elektrodenpaars näher an der ersten Elektrode 21₂ des zweiten Elektrodenpaars als an der ersten Elektrode 21₁ des ersten Elektrodenpaars angeordnet.

Figur 5 veranschaulicht eine Modifikation des Nervenstimulationsgeräts 1 gemäß Figur 4. Bei dem in Figur 5 dargestellten Nervenstimulationsgerät 1 besitzen der erste Stimulator 11 und der zweite Stimulator 12 eine gemeinsame zweite Elektrode 22. Diese gemeinsame zweite Elektrode 22 ist sowohl an die erste Signalquelle 31 als auch an die zweite Signalquelle 32 angeschlossen.

Figur 6 veranschaulicht eine Modifikation des in Figur 5 dargestellten Nervenstimulationsgeräts 1. Bei dem in Figur 6 dargestellten Nervenstimulationsgerät weisen der erste Stimulator 11 und der zweite Stimulator 12 eine gemeinsame Signalquelle 30 auf, die entsprechend der zuvor erläuterten ersten und zweiten Signalquelle 31, 32 eine steuerbare Stromquelle 50 und eine Steuerschaltung 60 aufweist. Diese Signalquelle 30 ist sowohl zwischen die erste Elektrode 21₁ des ersten Stimulators 11 und die gemeinsame zweite Elektrode 22 als auch zwischen die erste Elektrode 21₂ des zweiten Stimulators 12 und die gemeinsame zweite Elektrode 22 geschaltet. Bei diesem Nervenstimulationsgerät 1 erfolgt die Erzeugung des ersten Reizes durch den ersten Stimulator 11 und des zweiten Reizes durch den zweiten Stimulator 12 exakt synchron. Der bei diesem Nervenstimulationsgerät 1 durch die Signalquelle 30 bereitgestellte Strom I50 teilt sich auf die Elektrodenpaare des ersten und zweiten Stimulators 11, 12 auf. Diese Elektrodenpaare umfassen die erste Elektrode 21₁ und die gemeinsame zweite Elektrode 22 als erstes Elektrodenpaar und die erste Elektrode 21₂ und die gemeinsame zweite Elektrode 22 als zweites Elektrodenpaar.

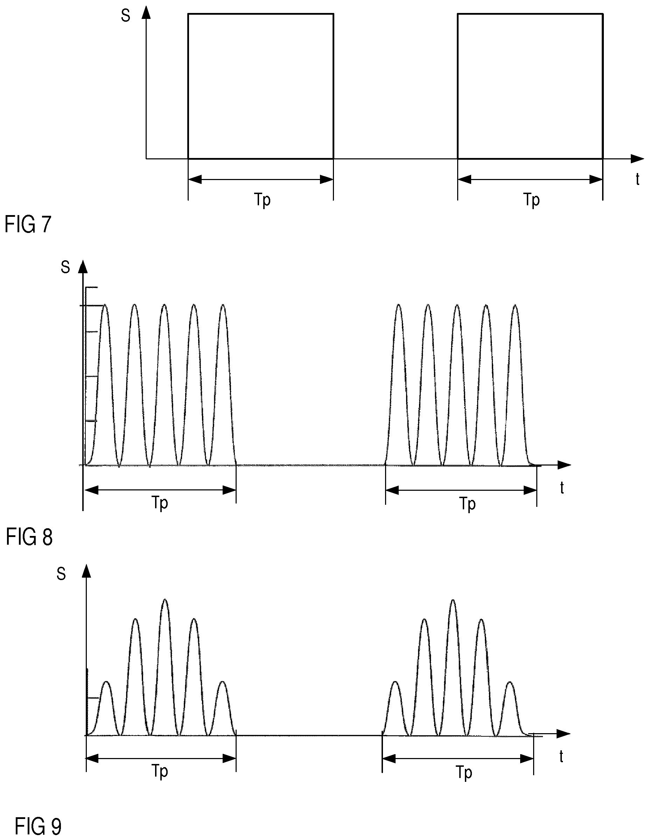

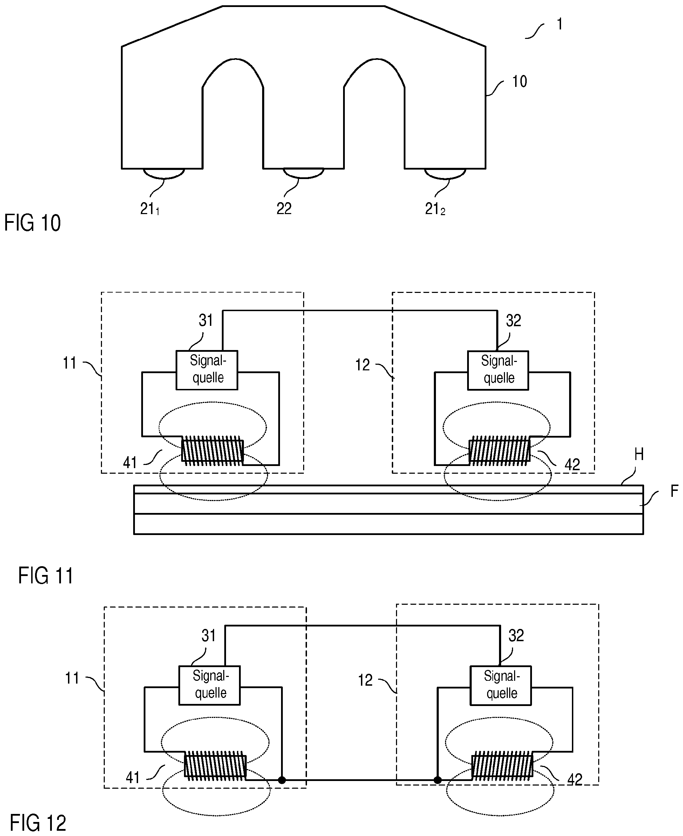

Figur 7 veranschaulicht einen Zeitverlauf eines Stimulationssignals S zur Beeinflussung einer Nervenfaser F gemäß einem Ausführungsbeispiel. Dieses Stimulationssignal S weist wenigstens einen Stimulationsimpuls auf, der im dargestellten Ausführungsbeispiel ein Rechteckimpuls ist. Bei den anhand der Figuren 3 bis 6 dargestellten Ausführungsbeispielen, bei denen ein Reiz durch Einprägen eines Stromes in die Nervenfaser F bzw. das umliegende Gewebe ausgelöst wird, entspricht dem Stimulationsimpuls ein Stromimpuls einer der beiden Signalquellen 31, 32 (bei den Ausführungsbeispielen gemäß der Figuren 3 bis 5) oder einem Stromimpuls der gemeinsamen Signalquelle 30 (bei dem Ausführungsbeispiel gemäß Figur 6). Eine Dauer des Stimulationsimpulses beträgt beispielsweise zwischen 75 Mikrosekunden (µs) und 300 Mikrosekunden (µs). Im Falle eines Stromimpulses als Stimulationsimpuls beträgt die maximale Amplitude beispielsweise zwischen 1 Milliampere (mA) und 70 Milliampere (mA). Gemäß einem weiteren Beispiel beträgt die Dauer eines Stimulationsimpulses zwischen 0,005 Mikrosekunden (5 Nanosekunden) und 0,007 Mikrosekunden (7 Nanosekunden). Diese kürzeren Stimulationsimpulse eignen sich insbesondere für eine Vorrichtung, wie sie in den Figuren 11-13 dargestellt ist und nachfolgend noch erläutert wird. Die Amplitude des Stimulationsimpulses kann über die gesamte Dauer Tp des Stimulationsimpulses annähernd konstant sein (wie in Figur 7 dargestellt ist). Dies ist jedoch lediglich ein Beispiel. Gemäß einem weiteren Ausführungsbeispiel variiert die Amplitude des Stimulationsimpulses über dessen Dauer Tp. Bei einem Ausführungsbeispiel ist vorgesehen, dass der Stimulationsimpuls die Form der positiven Halbwelle eines Sinussignals besitzt oder auf andere Weise zu Beginn des Stimulationsimpulses kontinuierlich ansteigt und gegen Ende des Stimulationsimpulses kontinuierlich absinkt.

Gemäß einem Ausführungsbeispiel ist vorgesehen, dass das Nervenstimulationsgerät 1 zur Reizung der Nervenfaser F mehrere zeitlich aufeinander folgende Stimulationsimpulse erzeugt. Eine Frequenz, mit der diese Stimulationsimpulse erzeugt werden, liegt beispielsweise zwischen 1 Hz und 50 Hz, insbesondere zwischen 3 Hz und 25 Hz oder zwischen 3 Hz und 15 Hz. Bei jeder Stimulation (Reizung) der Nervenfaser F durch das Nervenstimulationsgerät 1 werden in erläuterter Weise zwei in entgegengesetzte Richtung verlaufende Reize ausgelöst. Diese Reize blockieren sich gegenseitig, blockieren jedoch auch eine Reizausbreitung von einem inneren Organ zum Gehirn und umgekehrt, wodurch die eingangs erläuterten positiven Effekte erzielt werden können. Die Anzahl der insgesamt erzeugten Stimulationsimpulse ist abhängig von der Frequenz, mit der die Stimulationsimpulse erzeugt werden und der Behandlungsdauer. Die Behandlungsdauer beträgt beispielsweise zwischen 1 Minute und 20 Minuten.

Figur 8 veranschaulicht ein weiteres Ausführungsbeispiel eines Stimulationssignals S. Jeder Stimulationsimpuls dieses Stimulationssignals S ist ein oszillierendes Signal mit einer rechteckförmigen Einhüllenden. Die Oszillationsfrequenz des oszillierenden Signals innerhalb eines Impulses, d.h. unterhalb der Einhüllenden, beträgt beispielsweise zwischen 1 kHz und 15 kHz, insbesondere zwischen 4 kHz und 6 kHz. Gemäß einem weiteren Beispiel beträgt die Oszillationsfrequenz zwischen 100 MHz und 200 MHz. Dieser höhere Frequenzbereich eignet sich insbesondere für eine Vorrichtung, wie sie in den Figuren 11-13 dargestellt ist und nachfolgend noch erläutert wird. Für die Dauer des Stimulationsimpulses und gegebenenfalls eine Frequenz, mit der mehrere aufeinander folgende Stimulationsimpulse erzeugt werden, gelten die im Zusammenhang mit Figur 7 gemachten Erläuterungen entsprechend.

Gemäß einem Beispiel wird jeder Stimulationsimpuls so erzeugt, dass er 3 bis 7, insbesondere 3 bis 5 Impulse mit der Oszillationsfrequenz umfasst, die mit der oben genannten Oszillationsfrequenz erzeugt werden. Die Dauer Tp eines Stimulationsimpulses ergibt sich dann aus der Anzahl der Impulse multipliziert mit der Dauer eines Impulses, wobei die Dauer eines Impulses dem Kehrwert der Oszillationsfrequenz entspricht.

Figur 9 zeigt ein weiteres Ausführungsbeispiel eines Stimulationssignals. Bei diesem Ausführungsbeispiel ist der wenigstens eine Stimulationsimpuls ebenfalls ein oszillierendes Signal, und zwar mit einer Halbwelle eines Sinussignals als Einhüllende.

Selbstverständlich ist die Form des Stimulationsimpulses gemäß der Figuren 7-9 (wobei bei den Figuren 8-9 die Form des Stimulationsimpulses durch die Einhüllende bestimmt ist) nicht auf eine Rechteckform (vgl. Figuren 7-8) oder die Form einer Sinushalbwelle (vgl. Figur 9) beschränkt sondern andere Formen sind ebenfalls möglich. So ist gemäß einem Beispiel der Stimulationsimpuls dreieckförmig (zackenförmig) oder nur teilweise sinusförmig, d.h. er besitzt beispielsweise nur eine sinusförmige Flanke und eine Flanke, die nicht sinusförmig ist, wie beispielsweise eine rechteckförmige Flanke.

Das Stimulationssignal ist gemäß einem Beispiel (wie in den Figuren 7-9 dargestellt) ein Gleichsignal, d.h. der Signalpegel der einzelnen Stimulationsimpulse variiert nur zwischen Null und einem durch die Signalform vorgegebenen maximalen Amplitudenwert.

Bei einem weiteren Ausführungsbeispiel ist vorgesehen, dass die Elektroden des ersten und zweiten Elektrodenpaars des ersten und zweiten Stimulators 11, 12 keine elektrisch leitenden Oberflächen, sondern elektrisch isolierende Oberflächen besitzen. Zur Auslösung eines Reizes in der Nervenfaser F werden die Elektrodenpaare, in entsprechender Weise wie zuvor anhand von Figur 4 erläutert, in Kontakt mit der Haut H gebracht, oder zumindest in die Nähe der Haut H gebracht. Anstelle des Stromimpulses wird in diesem Fall allerdings ein Spannungsimpuls zwischen die Elektroden jedes Elektrodenpaares angelegt. Jeder dieser Spannungsimpulse bewirkt ein elektrisches Feld zwischen den Elektroden eines Elektrodenpaares, wobei dieses elektrische Feld eine Reizung der Nervenfaser F, das heißt die Auslösung eines Reizes, bewirkt. Die Spannungsimpulse sind dabei jeweils so zu polen, dass Elektronen in dem elektrischen Feld in Richtung der zweiten Elektroden 22₁, 22₂ bzw. in Richtung der gemeinsamen zweiten Elektrode 22 gezogen werden. Das heißt, das positivere Potenzial jedes Spannungsimpulses ist das elektrische Potenzial der zweiten Elektroden 22₁, 22₂ bzw. der gemeinsamen zweiten Elektrode 22.

Die Stimulationsimpulse sind bei diesem Ausführungsbeispiel Spannungsimpulse. Die zeitlichen Verläufe dieser Spannungsimpulse können den anhand der Figuren 7 bis 9 erläuterten zeitlichen Verläufen entsprechen.

Die zuvor erläuterten ersten und zweiten Stimulatoren 11, 12 können beispielsweise in einem Gehäuse untergebracht sein. Figur 10 zeigt schematisch ein solches Gehäuse 10, aus dem die Elektrodenpaare des ersten und zweiten Stimulators 11, 12 herausragen. Bei dem dargestellten Beispiel besitzen diese Elektrodenpaare eine gemeinsame zweite Elektrode 22, so dass insgesamt nur drei Elektroden vorhanden sind. Selbstverständlich könnte das Nervenstimulationsgerät jedoch auch mit vier Elektroden realisiert sein, wie dies zuvor anhand der Figuren 3 und 4 erläutert wurde. Eine Variante mit zwei Elektroden ist ebenso denkbar (nur in eine Richtung ohne Blockade).

Figur 11 veranschaulicht ein weiteres Ausführungsbeispiel des Nervenstimulationsgeräts 1. Bei diesem Ausführungsbeispiel besitzen die beiden Stimulatoren 11, 12 jeweils einen Elektromagnet 41, 42, der durch eine Signalquelle 31, 32 angesteuert ist. Die Signalquellen 31, 32 können beispielsweise Stromquellen sein, wie sie anhand von Figur 4 erläutert wurden. Jeder der beiden Elektromagnete 41, 42 ist in der Lage, ein magnetisches Feld zu erzeugen, wenn er von einem Strom durchflossen ist. Ein solches magnetisches Feld ist in gleicher Weise wie der zuvor erläuterte Strom oder das elektrische Feld in der Lage, einen Reiz in einer Nervenfaser F auszulösen. Zur Reizung einer Nervenfaser F mit dem Ziel einer Blockade einer Reizweiterleitung in der Nervenfaser F wird das Nervenstimulationsgerät 1 derart auf die Haut H aufgesetzt oder in die Nähe der Haut H gebracht, dass das magnetische Feld eine Depolarisation der Nervenfaser F, und damit eine Reizauslösung bewirken kann. Die Magnetfelder können dabei insbesondere den lonenaustausch über die Wand der Nervenfaser, der für eine Reizausbreitung notwendig ist, unterbrechen oder eine Reizausbreitung in entgegengesetzten Richtungen hervorrufen. Ein Stimulationsimpuls zur Auslösung eines Reizes in der Nervenfaser F ist bei diesem Ausführungsbeispiel ein elektromagnetischer Impuls, der durch einen der beiden Elektromagnete 41, 42 erzeugt wird. Hervorgerufen wird dieser elektromagnetische Impuls durch einen Stromimpuls der jeweiligen Signalquelle 31, 32. Diese Stromimpulse werden so erzeugt, dass das durch den ersten Elektromagnet und den zweiten Elektromagnet erzeugte Magnetfeld entgegengesetzte Feldrichtungen besitzen.

Figur 12 zeigt eine Modifikation des in Figur 11 dargestellten Nervenstimulationsgeräts. Bei dem in Figur 11 dargestellten Ausführungsbeispiel besitzen die beiden Elektromagnete 41, 42 einen gemeinsamen Anschluss.

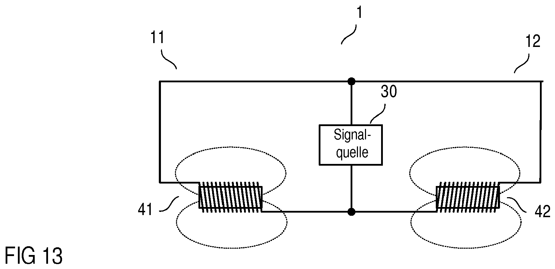

Figur 13 zeigt eine weitere Modifikation. Bei diesem Ausführungsbeispiel besitzen die beiden Stimulatoren 11, 12 eine gemeinsame Signalquelle 30, die den ersten Elektromagnet 41 und den zweiten Elektromagnet 42 versorgt. Bei diesem Ausführungsbeispiel werden die gegenläufigen Reize exakt synchron erzeugt. Bei einem weiteren Ausführungsbeispiel (nicht dargestellt) ist vorgesehen, dass die Vorrichtung mehr als zwei Stimulatoren mit je einem Elektromagnet aufweist, wobei gemäß einem Beispiel zwei unmittelbar benachbarte Elektromagnete jeweils Magnetfelder mit entgegengesetzten Feldrichtungen erzeugen.

Die zuvor erläuterten Vorrichtungen weisen jeweils zwei Stimulatoren auf, die je eine Signalquelle oder die eine gemeinsame Signalquelle aufweisen. Diese wenigstens eine Signalquelle der Vorrichtung erzeugt gemäß einem Beispiel eine Gleichsignal. Mit der erläuterten Vorrichtung lässt sich eine vollständige Blockade der stimulierten Nervenfaser, wie beispielsweise einer C-Faser des Vagusnervs erreichen.

Eine Blockade einer Reizausbreitung in nur einer Richtung lässt sich durch eine Vorrichtung erreichen, die nur einen der zuvor erläuterten Stimulatoren aufweist, wenn dieser Stimulator Reizerzeugungsmittel mit einer Gleichsignalquelle aufweist, wobei diese Gleichsignalquelle Stimulationsimpulse in der zuvor erläuterten Weise erzeugt. Die Reizerzeugungsmittel können wie erläutert elektrisch leitende Elektroden aufweisen, die geeignet sind Gleichstromimpulse abzugeben, elektrisch isolierte Elektroden aufweisen, die geeignet sind ein gleichgerichtetes elektrisches Feld zu erzeugen, oder einen Elektromagnet aufweisen, der geeignet ist, ein gleichgerichtetes magnetisches Feld zu erzeugen. Im Übrigen gelten die zuvor für die beiden Stimulatoren 11, 12 gemachten Ausführungen für den einen Stimulator in entsprechender Weise.

## Patentansprüche

1. Nervenstimulationsgerät, das aufweist:
einen ersten Stimulator (11), der dazu ausgebildet ist, zeitlich aufeinanderfolgend mehrere erste Reize an einer ersten Position (P1) in einer Nervenfaser (F) eines Nervs eines Menschen oder eines Säugetiers auszulösen, die sich nur in einer ersten Richtung (R1) in der Nervenfaser ausbreiten;
einen zweiten Stimulator (12 ), der dazu ausgebildet ist, zeitlich aufeinanderfolgend mehrere zweite Reize an einer zweiten Position (P2) in der Nervenfaser (F) auszulösen, die sich nur in einer zweiten Richtung (R2), die der ersten Richtung (R1) entgegengesetzt ist, in der Nervenfaser (F) ausbreiten,
wobei die zweite Position (P2) in der ersten Richtung (R1) zu der ersten Position (P1) beabstandet ist, und
wobei der erste Stimulator (11) und der zweite Stimulator (12) derart synchronisiert sind, dass jeweils ein erster Reiz und ein zweiter Reiz innerhalb eines vorgegebenen Zeitfensters erzeugt werden, so dass sich die ersten und zweiten Reize gegenseitig in der Nervenfaser blockieren,
wobei der erste Stimulator (11) erste Reizerzeugungsmittel und der zweite Stimulator (12) zweite Reizerzeugungsmittel aufweist, die jeweils dazu ausgebildet sind, einen Reiz in die Nervenfaser einzukoppeln,
wobei die ersten und zweiten Reizerzeugungsmittel jeweils eine erste Reizelektrode (21₁, 22₁) und eine zweite Reizelektrode (21₂, 22₂) aufweisen,
wobei die erste Reizelektrode (21₁, 22₁) und die zweite Reizelektrode (21₂, 22₂) jeweils räumlich zueinander beabstandet sind, und
wobei die zweite Reizelektrode (22₁) des ersten Reizerzeugungsmittels und die zweite Reizelektrode (22₂) des zweiten Reizerzeugungsmittels durch eine gemeinsame Elektrode (22) gebildet sind.

2. Nervenstimulationsgerät nach Anspruch 1, bei dem die ersten und zweiten Reizelektroden (21₁, 22₁, 21₂, 22₂) der ersten und zweiten Reizerzeugungsmittel elektrisch leitende Oberflächen oder elektrisch isolierende Oberflächen besitzen.

3. Nervenstimulationsgerät nach Anspruch 1 oder 2, das wenigstens eine elektrische Signalquelle (31, 32; 30) aufweist, die dazu ausgebildet ist, ein Stimulationssignal (S), das wenigstens einen Stimulationsimpuls aufweist, für wenigstens eines der ersten und zweiten Reizerzeugungsmittel zu erzeugen.

4. Nervenstimulationsgerät nach Anspruch 3, das weiterhin aufweist:
eine erste elektrische Signalquelle (31), die an die ersten Reizerzeugungsmittel gekoppelt ist, und
eine zweite elektrische Signalquelle (32), die an die zweiten Reizerzeugungsmittel gekoppelt ist,
wobei die erste elektrische Signalquelle (31) und die zweite elektrische Signalquelle (32) derart synchronisiert sind, dass jeweils ein erster und ein zweiter Reiz innerhalb des vorgegebenen Zeitfensters erzeugt werden.

5. Nervenstimulationsgerät nach Anspruch 3 oder 4, bei dem die wenigstens eine Signalquelle dazu ausgebildet ist, den wenigstens einen Stimulationsimpuls mit einer der nachfolgenden Signalformen zu erzeugen:
als Rechteckimpuls,
als sinusförmigen Impuls,
als wenigstens teilweise sinusförmigen Impuls,
als oszillierendes Signal mit einer rechteckförmigen Einhüllenden,
als oszillierendes Signal mit einer wenigstens teilweise sinusförmigen Einhüllenden,
als oszillierendes Signal mit einer dreieckförmigen Einhüllenden.

6. Nervenstimulationsgerät nach Anspruch 5, bei dem eine Frequenz des als oszillierendes Signal ausgebildeten Stimulationsimpulses zwischen 1kHz und 15kHz, zwischen 4 kHz und 6kHz oder zwischen 100 MHz und 200 MHz beträgt.

7. Nervenstimulationsgerät nach einem der Ansprüche 3 bis 6, bei dem die wenigstens eine Signalquelle dazu ausgebildet ist, das Stimulationssignal mit mehreren aufeinanderfolgenden und zeitlich beabstandeten Stimulationsimpulsen zu erzeugen.

8. Nervenstimulationsgerät nach Anspruch 7, bei dem eine Frequenz, mit der die Stimulationsimpulse erzeugt werden, zwischen 1 Hz und 50 Hz, zwischen 3 Hz und 25 Hz oder zwischen 3 Hz und 15 Hz beträgt.

9. Nervenstimulationsgerät nach einem der Ansprüche 3 bis 8, bei dem die wenigstens eine Signalquelle dazu ausgebildet ist, das Stimulationssignal so zu erzeugen, dass eine Dauer des wenigstens einen Stimulationsimpulses zwischen 75 Mikrosekunden (µs) und 900 Millisekunden (ms) oder zwischen 0,005 Mikrosekunden und 0,007 Mikrosekunden beträgt.

10. Nervenstimulationsgerät nach einem der Ansprüche 3 bis 9, bei dem die wenigstens eine Signalquelle dazu ausgebildet ist, das Stimulationssignal so zu erzeugen, dass der wenigstens eine Stimulationsimpuls ein Stromimpuls ist.

11. Nervenstimulationsgerät nach einem der Ansprüche 3 bis 9, bei dem die wenigstens eine Signalquelle dazu ausgebildet ist, das Stimulationssignal so zu erzeugen, dass der wenigstens eine Stimulationsimpuls ein Spannungsimpuls ist.

## Claims

1. A nerve stimulation appliance, having:
a first stimulator (11) which is designed to sequentially trigger several first stimuli at a first position (P1) in a nerve fibre (F) of a nerve of a human being or a mammal which only propagate in a first direction (R1) in the nerve fibre;
a second stimulator (12) which is designed to sequentially trigger several second stimuli at a second position (P2) in the nerve fibre (F) which only propagate in a second direction (R2), which is opposite to the first direction (R1), in the nerve fibre (F),
wherein the second position (P2) is spaced from the first position (P1) in the first direction (R1), and
wherein the first stimulator (11) and the second stimulator (12) are synchronised such that a first stimulus and a second stimulus, respectively, are generated within a predetermined time window so that the first and the second stimuli block each other in the nerve fibre,
wherein the first stimulator (11) has first stimulus generating means and the second stimulator (12) has second stimulus generating means which are each designed to couple a stimulus into the nerve fibre,
wherein the first and the second stimulus generating means each have a first stimulus electrode (21₁, 22₁) and a second stimulus electrode (21₂, 22₂),
wherein the first stimulus electrode (21₁, 22₁) and the second stimulus electrode (21₂, 22₂) are each spatially spaced from one another, and
wherein the second stimulus electrode (22₁) of the first stimulus generating means and the second stimulus electrode (22₂) of the second stimulus generating means are formed by a common electrode (22).

2. The nerve stimulation appliance according to claim 1, wherein the first and the second stimulus electrodes (21₁, 22₁, 21₂, 22₂) of the first and the second stimulus generating means possess electrically conductive surfaces or electrically insulating surfaces.

3. The nerve stimulation appliance according to claim 1 or 2, which has at least one electrical signal source (31, 32; 30) which is designed to generate a stimulation signal (S), which has at least one stimulation pulse, for at least one of the first and the second stimulus generating means.

4. The nerve stimulation appliance according to claim 3, further having:
a first electrical signal source (31) which is coupled to the first stimulus generating means, and
a second electrical signal source (32) which is coupled to the second stimulus generating means,
wherein the first electrical signal source (31) and the second electrical signal source (32) are synchronised such that a respective first and second stimulus are generated within the predetermined time window.

5. The nerve stimulation appliance according to claim 3 or 4, wherein the at least one signal source is designed to generate the at least one stimulation pulse with one of the following signal forms:
as a rectangle pulse,
as a sinusoidal pulse,
as an at least partially sinusoidal pulse,
as an oscillating signal with a rectangular envelope,
as an oscillating signal with an at least partially sinusoidal envelope,
as an oscillating signal with a triangular envelope.

6. The nerve stimulation appliance according to claim 5, wherein a frequency of the stimulation pulse designed as an oscillating signal is between 1 kHz and 15 kHz, between 4 kHz and 6 kHz or between 100 MHz and 200 MHz.

7. The nerve stimulation appliance according to any one of claims 3 to 6, wherein the at least one signal source is designed to generate the stimulation signal with several consecutive and time-spaced stimulation pulses.

8. The nerve stimulation appliance according to claim 7, wherein a frequency with which the stimulation pulses are generated is between 1 Hz and 50 Hz, between 3 Hz and 25 Hz or between 3 Hz and 15 Hz.

9. The nerve stimulation appliance according to any one of claims 3 to 8, wherein the at least one signal source is designed to generate the stimulation signal such that a duration of the at least one stimulation pulse is between 75 microseconds (µs) and 900 milliseconds (ms) or between 0.005 microseconds and 0.007 microseconds.

10. The nerve stimulation appliance according to any one of claims 3 to 9, wherein the at least one signal source is designed to generate the stimulation signal such that the at least one stimulation pulse is a current pulse.

11. The nerve stimulation appliance according to any one of claims 3 to 9, wherein the at least one signal source is designed to generate the stimulation signal such that the at least one stimulation pulse is a voltage pulse.

## Revendications

1. Appareil de stimulation nerveuse comprenant :
un premier stimulateur (11), qui est conçu pour déclencher successivement plusieurs premiers stimulus à une première position (P1) dans une fibre nerveuse (F) d'un nerf d'un être humain ou d'un mammifère, qui se propagent uniquement dans une première direction (R1) dans la fibre nerveuse ;
un second stimulateur (12), qui est conçu pour déclencher successivement plusieurs seconds stimulus à une seconde position (P2) dans la fibre nerveuse (F), qui se propagent uniquement dans la fibre nerveuse (F) dans une seconde direction (R2), qui est opposée à la première direction (R1),
dans lequel la seconde position (P2) est espacée dans la première direction (R1) de la première position (P1), et
dans lequel le premier stimulateur (11) et le second stimulateur (12) sont synchronisés de manière à ce qu'un premier stimulus et un second stimulus soient respectivement générés dans une fenêtre de temps prédéterminée, de sorte que les premiers et seconds stimulus se bloquent mutuellement dans la fibre nerveuse,
dans lequel le premier stimulateur (11) a des premiers moyens de génération de stimulus et le second stimulateur (12) a des seconds moyens de génération de stimulus, qui sont chacun conçus pour coupler un stimulus dans la fibre nerveuse,
dans lequel les premiers et seconds moyens de génération de stimulus comprennent chacun une première électrode de stimulus (21₁, 22₁) et une seconde électrode de stimulus (21₂, 22₂),
dans lequel la première électrode de stimulus (21₁, 22₁) et la seconde électrode de stimulus (21₂, 22₂) sont respectivement spatialement espacées l'une de l'autre, et
dans lequel la seconde électrode de stimulus (22₁) du premier moyen de génération de stimulus et la seconde électrode de stimulus (22₂) du second moyen de génération de stimulus sont formées par une électrode (22) commune.

2. Appareil de stimulation nerveuse selon la revendication 1, dans lequel les première et seconde électrodes de stimulus (21₁, 22₁, 21₂, 22₂) des premiers et seconds moyens générateurs de stimulus présentent des surfaces électriquement conductrices ou des surfaces électriquement isolantes.

3. Appareil de stimulation nerveuse selon la revendication 1 ou 2, qui comprend au moins une source de signal électrique (31, 32 ; 30) qui est conçue pour générer un signal de stimulation (S), qui comprend au moins une impulsion de stimulation, pendant au moins l'un des premiers et seconds moyens de génération de stimulus.

4. Appareil de stimulation nerveuse selon la revendication 3, comprenant en outre :
une première source de signal électrique (31) couplée au premier moyen de génération de stimulus, et
une seconde source de signal électrique (32) couplée au second moyen de génération de stimulus,
dans lequel la première source de signal électrique (31) et la seconde source de signal électrique (32) sont synchronisées de manière à ce qu'un premier et un second stimulus soient respectivement générés dans la fenêtre temporelle prédéterminée.

5. Appareil de stimulation nerveuse selon la revendication 3 ou 4, dans lequel l'au moins une source de signal est conçue pour générer l'au moins une impulsion de stimulation avec l'une des formes de signal suivantes :
sous forme d'impulsion carrée,
sous forme d'impulsion sinusoïdale,
sous forme d'impulsion au moins partiellement sinusoïdale,
sous forme de signal oscillant à enveloppe rectangulaire,
sous forme de signal oscillant à enveloppe au moins partiellement sinusoïdale,
sous forme de signal oscillant à enveloppe triangulaire.

6. Appareil de stimulation nerveuse selon la revendication 5, dans lequel une fréquence de l'impulsion de stimulation conçue sous forme de signal oscillant est comprise entre 1 kHz et 15 kHz, entre 4 kHz et 6 kHz ou entre 100 MHz et 200 MHz.

7. Appareil de stimulation nerveuse selon l'une des revendications 3 à 6, dans lequel l'au moins une source de signal est conçue pour générer le signal de stimulation avec plusieurs impulsions de stimulation consécutives et espacées dans le temps.

8. Appareil de stimulation nerveuse selon la revendication 7, dans lequel une fréquence à laquelle les impulsions de stimulation sont générées est comprise entre 1 Hz et 50 Hz, entre 3 Hz et 25 Hz ou entre 3 Hz et 15 Hz.

9. Appareil de stimulation nerveuse selon l'une des revendications 3 à 8, dans lequel l'au moins une source de signal est conçue pour générer le signal de stimulation de sorte qu'une durée de l'au moins une impulsion de stimulation est comprise entre 75 microsecondes (µs) et 900 millisecondes (ms) ou entre 0,005 microseconde et 0,007 microseconde.

10. Appareil de stimulation nerveuse selon l'une des revendications 3 à 9, dans lequel l'au moins une source de signal est conçue pour générer le signal de stimulation de sorte que l'au moins une impulsion de stimulation est une impulsion de courant.

11. Appareil de stimulation nerveuse selon l'une des revendications 3 à 9, dans lequel l'au moins une source de signal est conçue pour générer le signal de stimulation de sorte que l'au moins une impulsion de stimulation est une impulsion de tension.
